# EUROPEAN PATENT SPECIFICATION

(11) **EP 2 402 045 B1**
(45) Date of publication and mention of the grant of the patent: **06.06.2018**
(21) Application number: 11171333.5
(22) Date of filing: 24.06.2011
(51) Int. Cl.: A61L 31/02, A61L 31/08, A61L 27/38, A61F 2/00

(54) **Medical product, in particular for management of tissue repair**
Medizinisches Produkt, insbesondere zur Verwaltung von Gewebereparatur
Produit médical, en particulier pour la gestion de réparation de tissus

(30) Priority: 25.06.2010 US 810649
(43) Date of publication of application: 04.01.2012
(73) Proprietor: Aesculap AG, 78532 Tuttlingen/Donau (DE)
(72) Inventor: Odermatt, Erich, 8200 Schaffhausen (CH); Friedrich, Volker, 08191 Barcelona/Rubi (ES); Casanovas Albalate, Marta, 08950 Esplugues de Llobregat, Barcelona (ES); Funk, Lutz, 08172 Sant Cugat del Vallés (ES)
(74) Representative: Patentanwälte Ruff, Wilhelm, Beier, Dauster & Partner mbB

(56) References cited:
- WO-A1-00/72782
- WO-A1-02/071931
- US-A1- 2008 161 837

## Description

The present invention relates to a medical product which is especially useful for tissue repair and also to a method for manufacturing the medical product.

Typical tissue repair devices include hernia repair devices and prolapse repair devices.

For example, a hernia mesh, which has the form of a textile net and comprises non-absorbable monofile threads of different thickness is described in the applicant's WO 2007/101630 A1.

In medicine, prolapse means the protrusion through the vagina or the rectum of organs such as a female pelvic organ, bladder or uterus, which may occur for only one of these organs or for more than one organ simultaneously. Prolapse is a fairly frequent problem in women and may in particular be the result of a difficult vaginal birth or a weakening of the connective tissues. A device that is useful for supporting the female pelvic organs is known from EP 1 549 245 B1.

Having regard to tissue repair devices, it is important to stress that these devices "per se" are recognized as foreign matters by the body of a patient, which may, by way of example, result in undesired inflammatory and in particular immunological responses. A suture material that addresses these problems in a biologically active way is disclosed in EP 1 634 608 A1.

US 2008/0161837 A1 discloses reinforced meshes for retropubic implants for treatment of urinary incontinence and/or pelvic floor disorders, wherein the meshes may comprise protruding barbs.
WO 00/72782 A1 discloses biocompatible, implantable tapes having a mesh structcure, which can be loaded with cells.
WO 02/071931 A1 discloses surgical meshes or slings comprising tangs.

In view of the foregoing, it is an object of the present invention to provide a medical product which circumvents disadvantages of tissue repair devices described in the prior art and particularly reduces the risk of inflammatory and in particular immunological reactions in the body of a patient.
This object is achieved by a medical product or medical device comprising at least one elongate body. The at least one elongate body comprises cells.

The term "at least one elongate body" as used herein may encompass one, i.e. a single, elongate body. However, more preferably, the term " at least one elongate body" means a plurality of elongate bodies, i.e. at least two or more elongate bodies. In other words, it is preferred according to the present invention that the medical product comprises a plurality of elongate bodies comprising cells. In this regard, it may be further within the scope of the present invention that each elongate body may comprise cells.
In a further embodiment, the medical product consists of or is made from a plurality of elongate bodies comprising cells.

The cells may partially or completely, in particular holohedrally, colonize or occupy the at least one elongate body. More specifically, the cells may partially or completely, in particular holohedrally, coat or layer the at least one elongate body.

Typically, the cells are present on, particularly coat or layer, the surface of the at least one elongate body. The term "surface" as used herein may encompass an interior and/or exterior surface of the at least one elongate body. By way of example, in view of at least one porous elongate body, the cells may be present on an exterior and/or interior surface of the at least one elongate body.
Further, the cells may be present within a lumen of the at least one elongate body. Further details are given in the following description. The at least one elongate body, comprises a cell-retaining structure.
The term "cell-retaining structure" as used herein is preferably understood as a structure that retains or withholds cells from becoming lost, in particular from being peeled off, during the implantation or insertion of the medical product into the body of a patient.

The cell-retaining structure advantageously allows for an effective population of cells to be delivered to a tissue repair zone together with the medical product.

Usually, the cell-retaining structure is present on the surface of the at least one elongate body.

The cell-retaining structure comprises cell-retaining elements designed as cell-capturing or cell-adhering elements.

Further, the cell-retaining elements may have a depth between 50 µm and 1 mm. Furthermore, the cell-retaining elements may be present on the surface of the at least one elongate body in a mutual distance between 100 µm and 3 mm.

The cell-retaining elements are designed as barbs and, thus allows for a better capturing and adhering of the cells.

In a further conceivable embodiment, the cell-retaining elements are present as erectable structures, which rest against the surface of the at least one elongate body. Thus, the cell-retaining elements may be erected, by way of example manually or by subjecting to external stimuli, thereby advantageously allowing for a directed cellular finishing of the at least one elongate body under the cell-retaining elements.
Advantageously, the cell-retaining elements may also serve as anchoring means for anchoring the at least one elongate body in biological tissues, particularly in human and/or animal tissues. Advantageously, the medical product, in particular the at least one elongate body, may be designed as self-anchoring medical product, in particular as at least one self-anchoring elongate body.

Generally, the cell-retaining elements may be present in different shapes and geometries. By way of example, the cell-retaining elements may be escutcheon-shaped, shield-shaped, scale-shaped, wedge-shaped, thorn-shaped, arrow-shaped, spike-shaped, twin-shaped, V-shaped, W-shaped, and, combinations thereof. Further, the cell-retaining elements are preferably pointed or tapered at their free ends. Furthermore, the cell-retaining elements may have a multi-tip configuration, in particular a twin-tip configuration. An example for cell-retaining elements having a twin-tip configuration is the above mentioned W-shaped formation of cell-retaining elements. Cell-retaining elements having a twin-tip configuration may in particular be based on flat cuts into the at least one elongate body, preferably formed with a small angular offset and in small intervals from each other.

Furthermore, the cell-retaining elements may be arranged in different dispositions on the surface of the at least one elongate body. More specifically, the cell-retaining elements may have a disposition onto the at least one elongate body that is selected from the group consisting of a row disposition, a staggered disposition, an overlapping disposition, an offset disposition, an offset and partially overlapping disposition, a zigzag disposition, random or arbitrary disposition, a meander-like disposition, a serpentine-like disposition, a sinus-like disposition, a spiral disposition, a helical disposition, and combinations thereof.

In a further embodiment, the cell-retaining elements may be unidirectionally or multidirectionally, in particular bidirectionally, arranged on the surface of the at least one elongate body.

The cell-retaining elements are designed as barbs protruding from the surface of the at least one elongate body.

The cells are present underneath the cell-retaining elements, which protrude from the surface of the at least one elongate body. More specifically, the cells are present onto surface areas, in particular cut surface areas, of the at least one elongate body, which are underneath the cell-retaining elements and which are preferably covered with the cell-retaining elements resting against the at least one elongate body's surface. As an alternative or in combination, the cells are present on the lower surface (underside) of the cell-retaining elements with the cell-retaining elements protruding from the surface of the at least one elongate body.

Advantageously, the cells are clamped or lodged between the cell-retaining elements and surface areas, in particular cut surface areas, of the at least one elongate body, which are underneath the cell-retaining elements and which are preferably covered with the cell-retaining elements resting against the at least one elongate body's surface. Thus, the risk of a cellular loss and/or loss of agents during implantation of the medical product and, if appropriate, during an initial wound healing may be effectively minimised.

More specifically, the cells may be attached to the cell-retaining elements and/or surface areas, in particular cut surface areas, of the at least one elongate body which are preferably underneath the cell-retaining elements.

In combination, depending on the nature of the at least one elongate body, the cells may be present within the at least one elongate body.

According to an especially preferred embodiment, the cells predominantly, in particular merely, colonize or occupy, in particular layer or coat, the cell-retaining structure , preferably cell-retaining elements thereof.

In a further embodiment, the cell-retaining structure, in particular cell-retaining elements thereof, is formed by means of injection moulding, extruding, stamping, pressing, cutting, laser, and the like. Preferably, the cell-retaining structure is formed by cuts made into the at least one elongate body. More preferably, the cell-retaining elements are designed as cuts into the at least one elongate body.

In a further embodiment, the cell-retaining elements are configured as breakthroughs, i.e. the cell-retaining elements are formed completely breaking through a wall of the at least elongate body.

In a further advantageous embodiment, the medical product may comprise elongate bodies comprising a cell-retaining structure and elongate bodies comprising no cell-retaining structure.

The term "cells" as used according to the invention encompasses at least one single cell. Preferably, the term "cells" encompasses a plurality, i.e. at least two cells, particularly a population of cells.
The term "agents" as used according to the invention encompasses at least one single agent. Preferably, the term "agent" encompasses a plurality of agents, i.e. at least two agents. Moreover, the term "agents" may refer to a combination of different agents.
The cells may form a homogeneous or heterogeneous population. Having regard to the different characteristics of different cell types, it may be preferable that the cells are present as a heterogeneous population. Thus, the at least one elongate body may advantageously be equipped or finished with different cellular characteristics.
In a preferred embodiment, the at least one elongate body is loaded, in particular inoculated, with the cells.
Typically, the cells are viable or living cells.
Generally, the cells may be autologous, alogenic, and/or xenogenic cells. However, in order to minimize the risk of inflammatory or immunological responses, it is especially preferred to use cells of autologous origin. In order to equip the at least one elongate body with autologous cells, the cells are typically harvested from a patient to be treated.
Preferably, the cells are somatic cells, in particular stromal cells, i.e. connective tissue cells. More preferably, the cells are derived from epithelial tissue, endothelial tissue, adipose tissue, chondral tissue, osseous tissue, cellular lineages, and combinations thereof.
In a preferred embodiment, the cells are stem cells, in particular mesenchymal stem cells. In principle, the cells may be embryonic and/or adult stem cells. However, in order to meet ethic concerns, adult stem cells may be preferred and embryonic stem cells may be preferably excluded.
In a preferred embodiment, the cells are pluripotent stem cells. The term "pluripotent stem cells" as used according to the invention relates to stem cells that are able to differentiate a variety of tissues.

Preferably, the stem cells are derived from adipose tissue, in particular liposuctioned fat, bone marrow, blood, dental pulp, cornea, undifferentiated cell lineages such as undifferentiated fibroblasts, and combinations thereof. Especially preferred are adipose tissue-derived mesenchymal stem cells, due to their easy obtention (either from liposuction or lipectomy), a low donor-site morbidity and a high cell yield.

In a further embodiment, the cells are engineered, in particular genetically engineered, cells. By way of example, the cells may be engineered to express characteristics of other cell types, preferably selected from the group consisting of epithelial cells, endothelial cells, chondrocytes, osteocytes, fibroblasts, adipocytes, miocytes, neurons, astrocytes, oligodentrocytes, hepatocytes, pancreatic cells, progenitor cells thereof, stem cells thereof, and combinations thereof.

More specifically, the cells may be engineered to secrete factors such as cellular and/or synthetic factors. The factors may be selected from the group consisting of antimicrobial, in particular antibiotic, factors, disinfecting factors, anti-inflammatory factors, wound healing promoting factors, cellular growth factors, morphogenetic factors, cytokines, peptides, proteins, extracellular components, cellular differentiating factors, cellular adhesion factors, cellular recruiting factors, anesthetic factors, and combinations thereof.

Useful growth factors may be selected from the group consisting of fibroblast growth factor (FGF), transforming growth factor (TGF), platelet derived growth factor (PDGF), epidermal growth factor (EGF), granulocyte-macrophage colony stimulation factor (GMCSF), vascular endothelial growth factor (VEGF), insulin-like growth factor (IGF), hepatocyte growth factor (HGF), interleucin-1B (IL-1B), interleucin-8 (IL-8), nerve growth factor (NGF), and combinations thereof.

Useful extracellular components may be selected from the group consisting of collagen, reticulin, elastin, vitronectin, fibronectin, lam-inin, mucopolysaccharides such as hyaluronic acid, salts thereof, and combinations thereof.

Further, the cells may be engineered, in particular genetically engineered, to carry medicinal or pharmaceutical, in particular therapeutic, agents such as chemotherapeutic agents and/or radiotherapeutic agents. Useful agents may be selected from the group consisting of cisplatin, carboplatin, paclitaxel, Iridium-192, lodine-125, and combinations thereof.

In an especially preferred embodiment, the cells are selected from the group consisting of epithelial cells, endothelial cells, chondrocytes, osteocytes, fibroblasts, adipocytes, miocytes, neurons, astrocytes, oligodentrocytes, hepatocytes, pancreatic cells, progenitor cells thereof, stem cells thereof, engineered, in particular genetically engineered, cells thereof, and combinations thereof.

The at least one elongate body comprises a coating. The coating may in particular seal openings such as holes, perforations or breakthroughs of the at least one elongate body. The coating preferably facilitates, enforces and/or enhances adherence of the cells. Preferably, the coating material comprises cell-adhesion factors. By way of example, the coating material may be selected from the group consisting of peptides, extracellular matrix proteins, in particular from eukaryote cells, antibodies, protein antigens, sugars, lipids, salts thereof, and combinations thereof. Useful peptides may be peptides having an amino acid sequence comprising arginine, glycine and aspartic acid. Useful extracellular proteins are selected from the group consisting of collagen, vitronectin, fibronectins, laminins, salts thereof, and combinations thereof. In order to equip the at least one elongate body with the coating, the at least one elongate body may be, by way of example, soaked or immersed into a broth of the coating material.

In a further embodiment, the coating may be present as a porous coating, in particular in the form of a foam. This beneficially contributes to an enlargement of the surface of the at least one elongate body allowing for more cells to be adhered thereto. A coating as described in this paragraph may be applied onto the thread by means of sheath extrusion, for example.

The at least one elongate body may comprise agents, in particular factors, additionally to cells. More specifically, the at least one elongate body may be equipped or finished, in particular loaded, with agents. This is particularly useful to confer the at least one elongate body desired characteristics, in particular in view of therapeutic terms. Useful agents may be agents that foster cellular colonization of the at least one elongate body. Further, useful agents may be agents that induce differentiation of stem cells and/or expression of cellular characteristics such as secretion of desired substances, for example peptides, proteins, cytokines, and the like.

Preferably, useful agents may be factors that are selected from the group consisting of antimicrobial, in particular antibiotic, factors, disinfecting factors, anti-inflammatory factors, wound healing promoting factors, cellular growth factors, morphogenetic factors, cytokines, peptides, proteins, extracellular components, cellular differentiating factors, cellular adhesion factors, cellular recruiting factors, anesthetic factors, and combinations thereof.

Useful growth factors may be selected from the group consisting of fibroblast growth factor (FGF), transforming growth factor (TGF), platelet derived growth factor (PDGF), epidermal growth factor (EGF), granulocyte-macrophage colony stimulation factor (GMCSF), vascular endothelial growth factor (VEGF), insulin-like growth factor (IGF), hepatocyte growth factor (HGF), interleucin-1 B (IL-1B), interleucin-8 (IL-8), nerve growth factor (NGF), and combinations thereof.

Useful extracellular components may be selected from the group consisting of collagen, reticulin, elastin, vitronectin, fibronectin, laminin, mucopolysaccharides such as hyaluronic acid, salts thereof, and combinations thereof.

In a further embodiment, the agents are medicinal or pharmaceutical, particularly therapeutic, agents such as chemotherapeutic agents and/or radiotherapeutic agents. Useful agents may be selected from the group consisting of cisplatin, carboplatin, paclitaxel, Iridium-192, lodine-125, and combinations thereof.

In a further embodiment, the at least one elongate body may be absorbable, partially absorbable or non-absorbable. Further, the at least one elongate body may be made of a natural and/or synthetic material.

In a preferred embodiment, the at least one elongate body is made of a non-absorbable material that is preferably selected from the group consisting of polyolefine such as polyethylene, polypropylene, polyvinylidene difluoride (PVDF), polytetrafluoroethylene (PTFE), in particular expanded polytetrafluoroethylene (ePTFE), polytetrafluoropropylene or polyhexafluoropropylene, polyester such as polyethylene terephthalate, polypropylene terephthalate or polybutylene terephthalate, polyamide such as nylon 6 or nylon 6.6, polyurethane, silk, cotton, copolymers thereof and combinations thereof. Especially preferred is nylon, silk, polyester, cotton or a mixture of polyester and cotton.

More specifically the at least one elongate body may be made of a polyethylene selected from the group consisting of low-density polyethylene (LDPE), high-density polyethylene (HDPE), high-molecular-weight polyethylene (HMWPE), ultra-high-molecular-weight polyethylene (UHMWPE), and combinations thereof.

In a further preferred embodiment, the at least one elongate body may be made of an absorbable material such as a polyhydroxyalkanoate. More specifically, the at least one elongate body may be made of an absorbable material that is preferably selected from the group consisting of polyglycolide, polylactide, polye-ε-caprolactone, polytrimethylene carbonate, polyparadioxanone, poly-3-hydroxybutyrate, poly-4-hydroxybutyrate, copolymers thereof and combinations thereof. A preferred copolymer is made of glycolide and lactide, in particular in a weight ratio from 9:1 to 1:9. A further preferred copolymer is made of glycolide and ε-caprolactone.

The term "copolymer" according to the invention is preferably understood as a polymer that is composed of at least two different monomer units. Thus, tripolymers, tetrapolymers, and the like may also be encompassed by the term "copolymer". More specifically, a copolymer referred to in this invention may be present as a random copolymer, alternating copolymer, block or segmented copolymer or graft copolymer. Further, the copolymer may have an isotactic, syndiotactic or atactic structure.

According to an especially preferred embodiment, the at least one elongate body is designed as a mass elongate body, in particular as a thread, strand or yarn. More specifically, the at least one elongate body may be designed as a fibre or monofilament, pseudo monofilament or multifilament, preferably as a braided or twisted multifilament.

In case that the medical device comprises a plurality of elongate bodies, it may be further within the scope of the present invention that the elongate bodies may be selected from the group consisting of fibres or monofilaments, pseudo monofilaments, multifilaments, in particular braided and/or twisted multifilaments, and combinations thereof.

Further, the at least one elongate body may be present in an undrawn or drawn state. However, in view of mechanical stability, in particular tensile strength, of the at least one elongate body, it is advantageous that the at least one elongate body is designed as a drawn elongate body. In this regard, it may be advantageously that the at least one elongate body comprises cell-retaining elements, which are formed, in particular cut, in the at least one elongate body when the at least one elongate body is present in an undrawn state, wherein the at least one elongate body is drawn after forming the cell-retaining elements. In other words, the at least one elongate body may be obtained or obtainable by a method comprising the steps of:
a) forming, in particular cutting, cell-retaining elements into at least one undrawn elongate body, and
b) drawing the at least one elongate body.

Further, the at least one elongate body may comprise a core-sheath-structure. In this regard, the cell-retaining structure, in particular cell-retaining elements thereof, are preferably derived from the sheath material.

As an alternative, it may be especially preferred that the at least one elongate body is designed as a hollow body, i.e. a body having a lumen that is encased by a wall of the body. In this case, it is especially preferred that the cells are present within the lumen of the hollow body.

According to an especially preferred embodiment, the at least one elongate body is designed as a tube or hose. In this regard, it may be further within the scope of the present invention that the medical product consists or is made of one elongate body. In other words, it may be preferable that the medical product is designed as a tube or hose. By way of example, the tube and hose, respectively may have a diameter at most of 1 mm. The wall thickness may be in particular the seventh part of the diameter.

In a further embodiment, the at least one elongate body, preferably in the form of a tube, may comprise a textile structure, in particular a thread, preferably a braided thread, within the lumen thereof. The textile structure may advantageously serve as an additional support structure for the cells and may in particular contribute to a retarded release thereof. Thus, undesired local in vivo accumulations of cells may be avoided.

In case that the at least one elongate body is present as a hollow body, in particular as described above, a filler material may be present within the hollow body. In view of useful filler materials, reference is preferably made in its entirety to the afore-described coating materials.

In case that the medical product comprises a plurality of elongate bodies it may be conceivable that some of the elongate bodies are designed as threads, strands, yarns, and the like and some other elongate bodies are designed as tubes or hoses.

In a further embodiment, the medical product, in particular a plurality of elongate bodies thereof, comprise a textile construction that is preferably selected from the group consisting of Atlas, filet, tulle, mesh and openwork construction.

It is especially preferred that the medical product, in particular a plurality of elongate bodies thereof, may be in the form of a textile fabric, in particular a textile area-measured material or a two-dimensional fabric. More specifically, the medical product, in particular a plurality of elongate bodies thereof, may be designed as a textile fabric that is selected from the group consisting of a woven fabric, a non-woven fabric, a felt or interlooped fabric, a knit fabric, in particular a warp knit fabric, preferably a formed-loop knit fabric, more preferably a formed-loop warp-knit fabric, a braided fabric, and combinations thereof.

In a further preferred embodiment, the medical product is designed as a textile mesh or textile net.

In a further embodiment, the medical product, in particular the at least one elongate body, may be present as a tape, particularly as a textile tape.

In an especially preferred embodiment, the medical product is designed as a medical implant, in particular a surgical implant, preferably being selected from the group consisting of hernia mesh, prolapse mesh, urine incontinence sling or mesh and hemostatic patch.

Further, the medical product may be designed as a medical wound closure device, in particular as a self-anchoring or knotless medical wound closure device.

Further, the medical product may be designed as a device for repair of cartilaginous defects, in particular of articular cartilaginous defects, preferably of meniscal defects.

A further aspect of the present invention encompasses a method for manufacturing a medical product according to the present invention. For this purpose, a medical product comprising at least one elongate body is finished or equipped with cells.

As an alternative, the present invention also relates to a method for manufacturing a medical product comprising a) finishing or equipping at least one elongate body with cells and b) processing the at least one elongate body to a medical product.

In general, the finishing may be performed before implantation of the medical product, in particular immediately before an operation. As an alternative or in combination, the finishing may be performed after implantation.
More preferably, the medical product, in particular the at least one elongate body, is loaded, preferably inoculated, with the cells.

In a further advantageous embodiment, the medical product, in particular the at least one elongate body, is incubated in the presence of a culturing medium including the cells. More specifically, the medical product, in particular the at least one elongate body, may be immersed in a culturing medium including the cells. As an alternative, a culturing medium including the cells may be inoculated onto the medical product, in particular onto the at least one elongate body.

For cellular colonization, the medical product, in particular the at least one elongate body, may be incubated in the presence of a culturing medium including the cells for 1 to 14 days, in particular 2 to 10 days, preferably 2 to 7 days.

The culturing medium may additionally include agents as described in the previous description.

After incubating, the medical product, particularly the at least one elongate body, may be washed, preferably in order to get rid of undesired components that may be in particular derived from a culturing medium.

The culturing medium may be present as a solution or suspension.

According to an especially preferred embodiment, the medical product, in particular the at least one elongate body, is immersed in a solution having a concentration of the cells which are enough to cover the medical product, in particular the at least one elongate body, for long enough for the cells to adsorb to the medical product, in particular to the at least one elongate body.

For a cellular finishing of the medical product, in particular of the at least one elongate body, it may be preferred to use a cell-containing solution or cell-containing suspension, preferably having a cell concentration of 50000 cells per ml.

Further, a cell-retaining structure, in particular cell-retaining elements thereof, of the medical product, in particular the at least one elongate body, may be coated, particularly precoated, with the cells . Depending on the nature of the cell-retaining elements, the coating may be performed within the cell-retaining elements, onto the cell-retaining elements or between the cell-retaining elements and surfaces of the at least one elongate body, which are preferably underneath the cell-retaining elements.

Further, the medical product, in particular the at least one elongate body, may be finished by aid of a solution or suspension including the cells. With respect to a tubular medical product, particularly with respect to at least one elongate body having a tubular-like form, the finishing is preferably achieved by filling the tubular lumen of the medical product, particularly of the at least one elongate body, with a solution or suspension of the cells. This may be advantageously achieved by means of a surgical cannula.

More specifically, for the manufacture of the medical product, at least one elongate body may be used having a tubular structure and having openings such as holes, perforations or breakthroughs (especially derived from cell-retaining elements that are designed as breakthroughs) in the wall of the at least one elongate body. The at least one elongate body is coated, advantageously with an absorbable material such as collagen, and the like in order to seal the openings. Afterwards, the at least one elongate body may be filled with the cells through the open ends of the at least one tubular elongate body. After filling, the open ends are preferably closed, by way of example, by applying heat. Finally, the medical product is implanted, with the cells being released through the openings with absorption of the coating and/or removal of the coating upon contact with body liquids and/or rinsing liquids.

With respect to further details and advantages, in particular in view of the medical device and in particular in view of the at least one elongate body, reference is made in its entirety to the previous description.

The features and advantages of the invention will become apparent from the following description of preferred embodiments and by reference to the examples in conjunction with the features of the dependent claims. Individual features can be realized either singly or severally in combination.

### 1. Materials

A tube made of polyparadioxanone (PDO) having an internal diameter of 0,5 mm and an external diameter of 0,8 mm and having barbs that were configured as breakthroughs, i.e. completely breaking through the wall of the tube, was used.

Further, human adult adipose tissue-derived stem cells and chondrocytes were used as cells for finishing the above mentionedtube.

### 2. Isolation and culturing of the cells

### 2.1. Isolation and culturing of adult human adipose tissue-derived stem cells

The adipose tissue was obtained by liposuction. A cannula with a blunt end was introduced into the subcutaneous space through a small peri-umbilical incision (less than 0.5 cm in diameter). The suction was performed by moving the cannula along the adipose tissue compartment located under the abdominal wall, thus aiding the mechanical disruption of the adipose tissue. To minimize the loss of blood, a saline and epinephrine solution was injected as a vasoconstriction agent. 80 to 100 ml of raw lipoaspirate cells were obtained from each patient using this procedure.

The lipoaspirate was placed in a flask containing sterile phosphate buffered saline (PBS) at pH 7.4 and 4°C. The resulting mixture was weighed using a precision scale. Afterwards, it was taken to a biological exposure chamber in order to be processed.

The adipose tissue was washed with PBS until all visible blood and aspirated liquids were eliminated. The step facilitated a more selective cell isolation, with less erythrocytes and debris, since the presence of these elements impaired subsequent steps in the process of isolating the adipose tissue-derived stem cells.

The remaining tissue was placed in a container and then stirred (using a magnetic stirrer) for 60 minutes at 37°C. After that, it was submitted to enzymatic digestion.

For enzymatic digestion, the following compounds (per gram of tissue) were used: 2 ml Dulbecco's Modified Eagle Medium (DMEM), 2 mg/ml Collagenase A, 20 mg/ml Bovine Serum Albumin (BSA) - Fraction V, 124 µg/ml penicillin and 100 µg/ml streptomycin.

After 60 minutes, the digestion was interrupted by the addition of Fetal Bovine Serum, in a proportion equal to 10 % of the total volume of the enzymatic solution. This inactivation of Collagenase was important to prevent cell lysis.

The digested material was transferred to a Falcon tube and centrifuged at 200 x g for 5 minutes.

After centrifugation, the floating fraction (which contained the adipocyte fraction) was discarded, and the pellet containing the stromal vascular fraction (SVF) was resuspended in a mixture of DMEM, 10 % FBS, 124 µg/ml penicillin and 100 µg/ml streptomycin.

The collected cells were placed in a 25 cm² culture bottle containing DMEM, 10 % FBS, 124 µg/ml penicillin and 100 µg/ml streptomycin. This mixture prevented bacterial contamination in the cell culture.

The culture bottles were left in an incubator, at a concentration of 5 % carbon dioxide and 37°C, in order to promote cell expansion.

During the first two days, the bottles were washed with PBS to remove debris, erythrocytes and other non-adherent cells (stem cells adhered to the bottom of the culture bottle, thus not being removed by the wash). By the end of this step, the resulting culture was almost exclusively constituted by adipose tissue-derived stem cells.

Once, the adipose tissue-derived stem cells were adequately isolated, the culture medium was changed on alternate days. Thus, the cells expanded and reached about 70 % confluence (which means cells grouped together and occupied about 70 % of the inner bottom surface of the culture bottle).

In this primary culture, once the cells reached the expected confluence, they were submitted to trypsinization (trypsin is a serine protease which was used to destroy the proteins that facilitate adhesion to the bottle and intercellularly), using 5 ml Trypsin-EDTA, thus forming a cell suspension.

That suspension was used to transfer cells to a larger bottle (75 cm²), with the purpose of increasing cell numbers.

Using the above described technique, around 1,000,000, adipose tissue-derived stem cells were obtained in 15 days, starting from 10 g of adipose tissue extract.

### 2.2 Isolation and culturing of chondrocytes

A cartilage biopsy was harvested arthroscopically from a patient's knee and transferred into a sterile flask containing a "transport medium" (DMEM/F12 with 20 % fetal calf serum). The flask containing the transport medium was delivered within 48 hours to the cell culturing laboratory, where the mixture was weighed using a precision scale and subsequently was taken to a biological exposure chamber, in order to be processed.

The chondrocytes were expanded in tissue culture flasks in a CO₂ incubator (5 % CO₂) at 37°C in growth medium for 3 to 6 weeks and later on maintained in DMEM/F12 with the patient's (mammal) own heat inactivated serum (range 10 to 20 %) for 3 to 10 days. The aforementioned growth medium was supplemented with L-ascorbic acid (50 µg/ml (300 µmol/l)), gentamicin sulfate (50 µg/ml (10 mmol/l)) and Fungizone (2 µg/ml (2.2 µmol /l)). When the chondrocyte culture had been expanded to the amount of cells needed for the repair of a cartilage lesion of a given patient (mammal), the cells were harvested by trypsinization in 0.25% trypsin in 1 mM EDTA, washed in medium containing fetal calf serum (20 %) and centrifuged at 900 x g for 10 minutes at room temperature, and resuspended to cell numbers between 0.5 to 2 x 10⁶ cells per 0.1 ml growth medium (5 to 20 x 10⁶ cells per 1 ml growth medium). The optimum cell count per 0.1 ml growth medium for implantation was around 1 million cells. In general, a cartilage defect has room for 0.1 ml cell suspension per 1 cm² defect.

### 3. Cellular finishing of the PDO-tube

### 3.1 Finishing of the tubes with adipose tissue-derived stem cells

The tube as recited under 1. Materials were cut into stripes having a length of approximately 1.1 cm and breadth of approximately 0.6 cm. Afterwards, the stripes were deposited onto square wells (12 x 12 mm) of a 12 well-plate. A suspension of adipose tissue-derived stem cells was prepared in a concentration of around 50,000 cells per ml and 1 ml of the suspension was added to each well. The culturing dishes onto which the stripes were deposited were cultured in the presence of the cellular suspension in an atmosphere with 5 % CO₂ at 37 °C for 24 hours.

Afterwards, the stripes were taken out of the wells and subsequently placed in wells containing DAPI/methanol-solution. The stripes were incubated in the presence of DAPI/methanol for 15 minutes at 37 °C. After incubation, the stripes were investigated under an electron microscope, thereby confirming a cellular colonization of the tube stripes on their interior and exterior surfaces. Having regard to the investigated barbed tube stripes, also the barbs were colonized by the cells.

### 3.2 Finishing of the tubes with chondrocytes

The tubes as recited under 1. Materials were cut into stripes having a length of approximately 1.1 cm and a breadth of approximately 0.6 cm. Afterwards, the stripes were deposited onto square wells (12 x 12 mm) of a 12 well-plate. A suspension of chondrocytes was prepared in a concentration of around 1 million cells per ml and 1 ml of the suspension was added to each well. The culturing dishes onto which the stripes were deposited were cultured in the presence of the cellular suspension in an atmosphere with 5 % CO₂ at 37 °C for 24 hours.

Afterwards, the stripes were taken out of the wells and subsequently placed in wells containing DAPI/methanol-solution. The stripes were incubated in the presence of DAPI/methanol for 15 minutes at 37 °C. After incubation, the stripes were investigated under an electron microscope, thereby confirming a cellular colonization of the tube stripes on their interior and exterior surfaces. Having regard to the investigated barbed tube stripes, also the barbs were colonized by the cells.

## Claims

1. Medical product, comprising at least one elongate body comprising a cell-retaining structure and cells, wherein the cell-retaining structure comprises cell-retaining elements, wherein the cells are present onto the at least one elongate body's surface areas underneath the cell-retaining elements and/or the cells are present onto the underside of the cell-retaining elements, **characterized in that** the cell-retaining elements are designed as barbs protruding from the surface of the at least one elongate body and the at least one elongate body further comprises a coating.

2. Medical product according to claim 1, **characterized in that** the cells are lodged between the cell-retaining elements and the at least one elongate body's surface areas underneath the cell-retaining elements.

3. Medical product according toclaim 1 or 2, **characterized in that** the cells are somatic cells, in particular stromal cells, preferably derived from epithelial tissue, endothelial tissue, adipose tissue, chondral tissue, osseous tissue, cornea, dental pulp, bone marrow, blood, cellular lineages, and combinations thereof.

4. Medical product according to one of the preceding claims, **characterized in that** the cells are stem cells, in particular adult stem cells, preferably mesenchymal stem cells, wherein the stem cells are preferably derived from adipose tissue, in particular liposuctioned fat, bone marrow, blood, cornea, dental pulp and/or undifferentiated cell lineages, in particular undifferentiated fibroblasts.

5. Medical product according to one of the preceding claims, **characterized in that** the cells are selected from the group consisting of epithelial cells, endothelial cells, chondrocytes, osteocytes, fibroblasts, adipocytes, miocytes, neurons, astrocytes, oligodentrocytes, hepatocytes, pancreatic cells, progenitor cells thereof, stem cells thereof, engineered, in particular genetically engineered, cells thereof, and combinations thereof.

6. Medical product according to one of the preceding claims, **characterized in that** the at least one elongate body is made of a non-absorbable material that is preferably selected from the group consisting of polyolefin such as polyethylene, polypropylene, polyvinylidene difluoride, polytetrafluoroethylene, in particular expanded polytetrafluoroethylene, polytetrafluoropropylene or polyhexafluoropropylene, polyester such as polyethylene terephthalate, polypropylene terephthalate or polybutylene terephthalate, polyamide such as nylon 6 or nylon 6.6, polyurethane, silk, cotton, copolymers thereof, and combinations thereof and/or of an absorbable material that is preferably selected from the group consisting of polyglycolide, polylactide, poly-ε-caprolactone, polytrimethylene carbonate, polyparadioxanone, poly-3-hydroxybu-tyrate, poly-4-hydroxybutyrate, copolymers thereof, and combinations thereof.

7. Medical product according to one of the preceding claims, **characterized in that** the at least one elongate body is designed as hollow body, preferably as a tube.

8. Medical product according to one of the preceding claims, **characterized in that** the medical product is selected from the group consisting of hernia mesh, prolapse mesh, urine incontinence mesh or sling or haemostatic device.

9. Method for manufacturing a medical product according to one of the preceding claims, **characterized in that** a medical product comprising at least one elongate body is finished with cells or at least one elongate body is finished with cells and subsequently is processed to a medical product.

## Patentansprüche

1. Medizinisches Produkt, umfassend zumindest einen langgestreckten Körper, der eine Zell-Retentionsstruktur und Zellen umfasst, wobei die Zell-Retentionsstruktur Zell-Retentionselemente umfasst, wobei die Zellen auf den Oberflächenbereichen des zumindest einen langgestreckten Körpers unter den Zell-Retentionselementen vorliegen und/oder die Zellen auf der Unterseite der Zell-Retentionselemente vorliegen,
**dadurch gekennzeichnet, dass**
die Zell-Retentionselemente als Widerhaken ausgebildet sind, die von der Oberfläche des zumindest einen langgestreckten Körpers abstehen und der zumindest eine langgestreckte Körper weiter eine Beschichtung umfasst.

2. Medizinisches Produkt nach Anspruch 1 **dadurch gekennzeichnet, dass** die Zellen zwischen den Zell-Retentionselementen und den Oberflächenbereichen des zumindest einen langgestreckten Körpers unter den Zell-Retentionselementen festgehalten sind.

3. Medizinisches Produkt nach Anspruch 1 oder 2, **dadurch gekennzeichnet, dass** die Zellen somatische Zellen, insbesondere Bindegewebszellen, sind und bevorzugt von Epithelgewebe, Endothelgewebe, adipösem Gewebe, Knorpelgewebe, Knochengewebe, Cornea, dentaler Pulpa, Knochenmark, Blut, Zelllinien und Kombinationen davon abgeleitet sind.

4. Medizinisches Produkt nach einem der vorhergehenden Ansprüche, **dadurch gekennzeichnet, dass** die Zellen Stammzellen, insbesondere adulte Stammzellen, bevorzugt mesenchymale Stammzellen sind, wobei die Stammzellen bevorzugt von adipösem Gewebe, insbesondere Liposuktionsfett, Knochenmark, Blut, Cornea, dentaler Pulpa und/oder undifferenzierten Zelllinien, insbesondere undifferenzierten Fibroblasten, abgeleitet sind.

5. Medizinisches Produkt nach einem der vorhergehenden Ansprüche, **dadurch gekennzeichnet, dass** die Zellen ausgewählt sind aus der Gruppe bestehend aus Epithelzellen, Endothelzellen, Chondrozyten, Osteozyten, Fibroblasten, Adipozyten, Myozyten, Neuronen, Astrozyten, Oligodendrozyten, Hepatozyten, Pankreaszellen, Progenitorzellen davon, Stammzellen davon, durch Zell-Engineering, insbesondere Gen-Engineering, erhaltene Zellen davon und Kombinationen davon.

6. Medizinisches Produkt nach einem der vorhergehenden Ansprüche, **dadurch gekennzeichnet, dass** der zumindest eine langgestreckte Körper ausgebildet ist aus einem nicht resorbierbaren Material, das bevorzugt ausgewählt ist aus der Gruppe bestehend aus Polyolefin, wie Polyethylen, Polypropylen, Polyvinylidendifluorid, Polytetrafluorethylen, insbesondere expandiertes Polytetrafluorethylen, Polytetrafluorpropylen oder Polyhexafluorpropylen, Polyester, wie Polyethylenterephthalat, Polypropylenterephthalat oder Polybutylenterephthalat, Polyamid, wie Nylon 6 oder Nylon 6,6, Polyurethan, Seide, Baumwolle, Copolymeren davon und Kombinationen davon und/oder aus einem resorbierbaren Material, das bevorzugt ausgewählt ist aus der Gruppe bestehend aus Polyglycolid, Polylactid, Poly-e-caprolacton, Polytrimethylencarbonat, Poly-para-dioxanon, Poly-3-hydroxybutyrat, Poly-4-hydroxybutyrat, Copolymeren davon und Kombinationen davon.

7. Medizinisches Produkt nach einem der vorhergehenden Ansprüche, **dadurch gekennzeichnet, dass** der zumindest eine langgestreckte Körper in Form eines Hohlkörpers, insbesondere eines Rohrs oder Schlauchs, ausgebildet ist.

8. Medizinisches Produkt nach einem der vorhergehenden Ansprüche, **dadurch gekennzeichnet, dass** das medizinische Produkt ausgewählt ist aus der Gruppe bestehend aus Hernien-Netz, Prolaps-Netz, Harninkontinenz-Netz oder -Band oder Hämostyptikum.

9. Verfahren zur Herstellung eines medizinischen Produkts nach einem der vorhergehenden Ansprüche, **dadurch gekennzeichnet, dass** ein medizinisches Produkt, das zumindest einen langgestreckten Körper umfasst, mit Zellen besiedelt wird oder zumindest ein langgestreckter Körper mit Zellen besiedelt wird und anschließend zu einem medizinischen Produkt verarbeitet wird.

## Revendications

1. Produit médical, comprenant au moins un corps allongé comprenant une structure de retenue de cellules et des cellules, dans lequel la structure de retenue de cellules comprend des éléments de retenue de cellules, dans lequel les cellules sont présentes sur les surfaces spécifiques de l'au moins un corps allongé en dessous des éléments de retenue des cellules et/ou les cellules sont présentes sur le revers des éléments de retenue de cellules, **caractérisé en ce que** les éléments de retenue de cellule sont conçus sous la forme de barbes dépassant de la surface de l'au moins un corps allongé et l'au moins un corps allongé comprend en outre un revêtement.

2. Produit médical selon la revendication 1, **caractérisé en ce que** les cellules sont logées entre les éléments de retenue de cellules et les surfaces spécifiques de l'au moins un corps allongé en dessous des éléments de retenue de cellules.

3. Produit médical selon la revendication 1 ou 2, **caractérisé en ce que** les cellules sont des cellules somatiques, en particulier des cellules stromales, de préférence dérivées d'un tissu épithélial, d'un tissu endothélial, d'un tissu adipeux, d'un tissu chondral, d'un tissu osseux, d'une cornée, d'une pulpe dentaire, d'une moelle osseuse, de sang, de lignées cellulaires et de combinaisons de ceux-ci.

4. Produit médical selon l'une quelconque des revendications précédentes, **caractérisé en ce que** les cellules sont des cellules souches, en particulier des cellules souches adultes, de préférence des cellules souches mésenchymateuses, les cellules souches étant de préférence dérivées d'un tissu adipeux, en particulier de graisse liposucée, de moelle osseuse, de sang, de cornée, de pulpe dentaire et/ou de lignées cellulaires indifférenciées, en particulier de fibroblastes indifférenciés.

5. Produit médical selon l'une quelconque des revendications précédentes, **caractérisé en ce que** les cellules sont sélectionnées dans le groupe constitué par des cellules épithéliales, des cellules endothéliales, des chondrocytes, des ostéocytes, des fibroblastes, des adipocytes, des myocytes, des neurones, des astrocytes, des oligodentrocytes, des hépatocytes, des cellules pancréatiques, des cellules progénitrices de ceux-ci, des cellules souches de ceux-ci, des cellules modifiées, en particulier modifiées génétiquement, de ceux-ci et des combinaisons de ceux-ci .

6. Produit médical selon l'une quelconque des revendications précédentes, **caractérisé en ce que** l'au moins un corps allongé est fait d'un matériau non résorbable qui est de préférence sélectionné dans le groupe constitué par une polyoléfine telle que le polyéthylène, le polypropylène, le difluorure de polyvinylidène, le polytétrafluoroéthylène, en particulier le polytétrafluoroéthylène expansé, le polytétrafluoropropylène ou le polyhexafluoropropylène, le polyester tel que le polyéthylène téréphtalate, le polypropylène téréphtalate ou le polybutylène téréphtalate, le polyamide tel que le nylon 6 ou le nylon 6.6, le polyuréthane, la soie, le coton, des copolymères de ceux-ci, et des combinaisons de ceux-ci et/ou d'un matériau résorbable qui est de préférence sélectionné dans le groupe constitué par le polyglycolide, le polylactide, la poly-ε-caprolactone, le polytriméthylène carbonate, la polyparadioxanone, le poly-3-hydroxybutyrate, le poly-4-hydroxybutyrate, des copolymères de ceux-ci, et des combinaisons de ceux-ci.

7. Produit médical selon l'une quelconque des revendications précédentes, **caractérisé en ce que** l'au moins un corps allongé est conçu en tant que corps creux, de préférence en tant que tube.

8. Produit médical selon l'une quelconque des revendications précédentes, **caractérisé en ce que** le produit médical est sélectionné dans le groupe constitué par une maille pour hernie, une maille pour prolapsus, une maille pour incontinence urinaire ou une bandelette ou un dispositif hémostatique.

9. Procédé de fabrication d'un produit médical selon l'une quelconque des revendications précédentes, **caractérisé en ce qu'**un produit médical comprenant au moins un corps allongé est finalisé avec des cellules ou au moins un corps allongé est finalisé avec des cellules et par la suite est transformé en produit médical.
